# EUROPEAN PATENT APPLICATION

(11) **EP 4 039 678 A1**
(43) Date of publication of application: **10.08.2022**
(21) Application number: 20872689.3
(22) Date of filing: 29.09.2020
(51) Int. Cl.: C07D 333/10, C07C 69/017, A61K 31/381

(54) **COMPOUND AND PREPARATION METHOD THEREFOR AND USE THEREOF**

(30) Priority: 30.09.2019 CN 201910945863; 30.09.2019 CN 201910944794; 30.09.2019 CN 201910945894
(71) Applicant: Shandong Luye Pharmaceutical Co., Ltd., Yantai, Shandong 264670 (CN)
(72) Inventor: ZHOU, Fengmei, Yantai, Shandong 264670 (CN); TAN, Wenjuan, Yantai, Shandong 264670 (CN); TANG, Qianqian, Yantai, Shandong 264670 (CN); WANG, Jinrong, Yantai, Shandong 264670 (CN); ZHANG, Rui, Yantai, Shandong 264670 (CN); YU, Fei, Yantai, Shandong 264670 (CN)
(74) Representative: Kreuels, Justus
(86) International application number: PCT/CN2020/118775
(87) International publication number: WO 2021/063347

(57) **Abstract**

Provided are a novel impurity compound of rotigotine behenate, rotigotine behenate or a preparation thereof containing less than 0.5wt% of the impurity compound and use of the impurity compound as a reference substance in the impurity detection of the rotigotine behenate or the preparation thereof.

## Description

### TECHNICAL FIELD

The present invention belongs to the field of pharmaceuticals, and particularly relates to a novel compound, a preparation method therefor and use thereof.

### BACKGROUND

The Parkinson's disease is a common neurodegenerative disease of middle-aged and old people. Dopamine receptor agonists are a class of important drugs for treating the Parkinson's disease. At present, clinically used dopamine receptor agonists include dopamine agonist drugs, such as rotigotine, pramipexole, ropinirole, pergolide and cabergoline.

Rotigotine behenate, disclosed in WO2018014277, is a 22-carbon saturated long-chain ester of rotigotine, which has the effects of long-time maintenance of effective plasma concentration, high bioavailability and long-acting stable release. The patent application discloses that saturated or unsaturated long-chain esters of rotigotine with more or less than twenty two carbons do not have the good effects mentioned above, with a structure shown below, and the esters are used for the adjuvant treatment of early secondary Parkinson's disease and advanced Parkinson's disease.

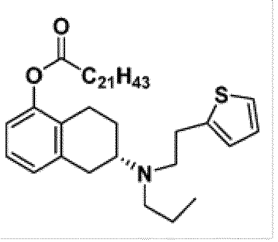

Impurities of a drug refer to substances present in the drug which have no therapeutic effect or affect the stability and therapeutic effect of the drug and are even harmful to the health of human bodies. There are two main sources of impurities: one is introduced in the process of production, including unreacted starting materials, chemical derivatives of impurities contained in the starting materials, synthesis byproducts and degradation products; and the other is generated due to a change in the physicochemical properties of a drug caused by the influence of external conditions during storage. Adverse reactions generated by the clinical use of a drug are not only related to the pharmacological activity of the drug but also sometimes closely related to impurities in the drug. Therefore, the standard study of impurities will directly affect the quality and safety of marketed drugs.

By knowing the chemical structure and synthetic route of impurities and identifying parameters that affect the content of impurities in a final product, the management of impurities in pharmaceutically active substances can be greatly enhanced. The monitoring of impurities in pharmaceutically active substances requires the establishment of quality standards and appropriate separation and detection conditions, so that impurities can be well-controlled. In quality standards, the impurity detection methods commonly employed at present mainly include liquid chromatography, etc.

### SUMMARY

The present invention aims to provide a novel compound, a preparation method therefor and use thereof.

The specific compounds provided by the present invention are as follows:

Compound I, chemical name: (*S*)-6-(2-(2-thienyl)ethyl)amino)-5,6,7,8-tetrahydronaphthalene-1-yl didodecanoate, having a structural formula shown as formula I:

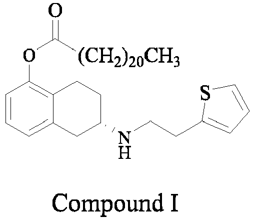

The compound I is a compound with an S configuration.

The compound I may be prepared by employing the following method: weighing (*S*)-6-(2-(thiophene-2-yl)ethylamino)-5,6,7,8-tetrahydronaphthalene-1-alcohol hydrobromide, triethylamine, and dichloromethane, and stirring the mixture at room temperature; adding di-*tert*-butyl dicarbonate, stirring the mixture at room temperature overnight, evaporating off the solvent under vacuum after the reaction is complete, stirring a sample with silica gel, and performing column chromatography to give an intermediate I; weighing the intermediate I, behenic acid, DMAP, and EDCI, adding toluene, uniformly stirring until the reaction is complete, adding an NH₄Cl solution, stirring, adding diatomite, performing suction filtration, allowing the solution to stand overnight, washing the solution with an NH₄Cl solution once, adding water into the organic phase, stirring, and after allowing the solution to stand, removing the aqueous phase; after the organic phase solvent is evaporated off, performing column chromatography to give an intermediate II; weighing and dissolving the intermediate II in dichloromethane, adding *N*-methylmorpholine, uniformly stirring, adding TMSI, and stirring the mixture at room temperature until the reaction is complete; evaporating off the organic solvent under vacuum, adding petroleum ether, stirring, performing suction filtration, concentrating and evaporating the mother solution, adding dichloromethane, and washing the solution with water twice; and concentrating, evaporating and drying the organic phase to give (*S*)-6-(2-(2-thienyl)ethyl)amino)-5,6,7,8-tetrahydronaphthalene-1-yl didodecanoate.

The NH₄Cl solution added in the aforementioned method is a 20% NH₄Cl solution.

The intermediate I, behenic acid, DMAP, EDCI and toluene weighed in the aforementioned method are stirred to react at 40 °C.

Di-*tert*-butyl dicarbonate added in the aforementioned method is slowly added dropwise. Compound II, chemical name: (*S*)-6-(propylamino)-5,6,7,8-tetrahydronaphthalene-1-yl didodecanoate, having a structural formula shown as formula II:

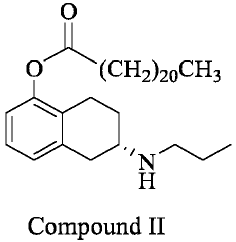

The compound II is a compound with an S configuration.

The compound II may be prepared by employing the following method: weighing (*S*)-6-(propylamino)-5,6,7,8-tetrahydronaphthalene-1-alcohol hydrobromide, triethylamine, and dichloromethane, and stirring the mixture at room temperature; adding di-*ter*t-butyl dicarbonate dropwise, stirring for 4 h, evaporating off the solvent under vacuum, and performing column chromatography to give an intermediate I; weighing the intermediate I, behenic acid, DMAP, EDCI, and toluene, and uniformly stirring until the reaction is complete; adding an NH₄Cl solution, stirring, adding diatomite, performing suction filtration, allowing the solution to stand overnight, washing the solution with an NH₄Cl solution once, adding water, stirring, and after allowing the solution to stand, removing the aqueous phase; after the organic phase solvent is evaporated off, performing column chromatography to give an intermediate II; weighing and dissolving the intermediate II in dichloromethane, adding *N*-methylmorpholine, uniformly stirring, then adding TMSI, and stirring the mixture at room temperature for reaction; evaporating off the organic solvent under vacuum, adding petroleum ether, stirring, performing suction filtration, and discarding the filter cake; concentrating and evaporating the mother solution, adding dichloromethane, and washing dichloromethane with water twice; and concentrating, evaporating and drying the organic phase to give (*S*)-6-(propylamino)-5,6,7,8-tetrahydronaphthalene-1-yl didodecanoate.

The NH₄Cl solution added in the aforementioned method is a 20% NH₄Cl solution.

The intermediate I, behenic acid, DMAP, EDCI and toluene weighed in the aforementioned method are stirred to react at 40 °C.

Di-*tert*-butyl dicarbonate added in the aforementioned method is slowly added dropwise.

Compound III, chemical name: compound 7,8-dihydronaphthalene-1-yl didodecanoate, having a structural formula shown as formula III.

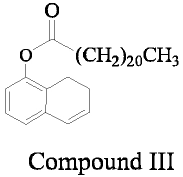

The compound III may be prepared by employing the following method: adding 5-hydroxy-1-tetralone, imidazole and TBS-Cl into DMF to form a light brown yellow solution, and reacting completely; pouring the reaction solution into water, precipitating a solid, performing filtration, washing the filter cake with water, then dissolving the filter cake in dichloromethane, washing the solution with water, adding petroleum ether after drying, passing through a silica gel pad to give 5-(*tert*-butyldimethylsiloxy)-3,4-dihydronaphthalene-1(2*H*)-one, dissolving 5-(*tert*-butyldimethylsiloxy)-3,4-dihydronaphthalene-1(2*H*)-one in tetrahydrofuran, adding methanol, adding sodium borohydride in batches, and reacting completely; concentrating the reaction solution, removing the organic solvent, extracting the product with ethyl acetate to give a light yellow oily substance, dissolving the light yellow oily substance in dichloromethane, adding a dichloromethane solution of 5-(*tert*-butyldimethylsiloxy)-1,2,3,4-tetrahydronaphthalene-1-ol and triethylamine into dichloromethane, adding a 600 mL dichloromethane solution of methylsulphonyl chloride dropwise while temperature is controlled, concentrating the reaction solution, adding water for dispersion, extracting the solution with *tert*-butyl methyl ether, concentrating the solution after drying, and performing silica gel column chromatography to give *tert*-butyl-(7,8-dihydronaphthalene-1-yloxy)dimethylsilane; and adding *tert*-butyl-(7,8-dihydronaphthalene-1-yloxy)dimethylsilane into methanol, adding cesium fluoride, reacting under the protection of nitrogen, concentrating the reaction solution after the reaction is complete, extracting the product with DCM after evaporation, then evaporating DCM, adding basic alumina, stirring a sample, passing through an basic alumina column (PE : DCM = 5 : 1) to give 7,8-dihydronaphthol, adding 7,8-dihydronaphthol into dichloromethane, adding triethylamine and a dichloromethane solution of behenoyl chloride, reacting completely, adding water into the reaction solution, stirring, and performing filtration to give the compound III, i.e., 7,8-dihydronaphthalene-1-yl didodecanoate.

The reaction under the protection of nitrogen in the aforementioned method is performed by heating to 60 °C.

In the aforementioned method, a dichloromethane solution of 5-(*tert*-butyldimethylsiloxy)-1,2,3,4-tetrahydronaphthalene-1-ol and triethylamine are added into dichloromethane, and the temperature is controlled to be less than 20 °C.

Any of the compounds I-III may be used as a reference sample for qualitative or quantitative determination of impurities of rotigotine behenate or a preparation thereof, and specifically, high performance liquid chromatography may be employed for determination. Any of the compounds I-III is dissolved in a solution to prepare a reference sample solution, and rotigotine behenate or a preparation thereof is dissolved in a solution to prepare a test sample solution. Analysis is performed by employing liquid chromatography to give a liquid chromatogram of the reference sample solution and the test sample solution. The peak times in the liquid chromatogram of the reference sample solution and the test sample solution are compared, it is determined that the test sample solution contains any of the compounds I-III, the peak areas of any of the compounds I-III in the liquid chromatogram of the reference sample solution and the test sample solution are compared, and the weight percentage of any of the compounds I-III in rotigotine behenate or the preparation thereof is determined according to an external standard method.

The chemical names corresponding to the abbreviations are as follows: DMAP: 4-dimethylaminopyridine; EDCI: 1-ethyl-3(3-dimethylpropylamine)carbodiimide; TMSI: iodotrimethylsilane; TBS-Cl: *tert*-butyldimethylsilyl chloride; DMF: *N*,*N*-dimethylformamide; DCM: dichloromethane; PE: petroleum ether; TLC: thin-layer chromatography; HPLC: high performance liquid chromatography; UPLC: ultra-high performance liquid chromatography.

Any of the compounds I-III of the present invention is at least 80% pure, preferably at least 90% pure, more preferably at least 95% pure, and most preferably at least 99% pure, as determined by HPLC or UPLC.

The present invention also provides a rotigotine behenate or a preparation thereof, which contains less than 0.5 wt% of any of the compounds I-III.

### BRIEF DESCRIPTION OF THE DRAWINGS

FIG. 1 is a high-resolution mass spectrogram of
   (*S*)-6-(2-(2-thienyl)ethyl)amino)-5,6,7,8-tetrahydronaphthalene-1-yl didodecanoate.
FIG. 2-1 is a UPLC chromatogram of a reference sample of (*S*)-6-(2-(2-thienyl)ethyl)amino)-5,6,7,8-tetrahydronaphthalene-1-yl didodecanoate.
FIG. 2-2 is a UPLC chromatogram comparing peak times of rotigotine behenate compound and a test sample of (*S*)-6-(2-(2-thienyl)ethyl)amino)-5,6,7,8-tetrahydronaphthalene-1-yl didodecanoate.
FIG. 3 is a high-resolution mass spectrogram of (*S*)-6-(propylamino)-5,6,7,8-tetrahydronaphthalene -1-yl didodecanoate.
FIG. 4-1 is a UPLC chromatogram of a reference sample of (*S*)-6-(propylamino)-5,6,7,8-tetrahydronaphthalene-1-yl didodecanoate.
FIG. 4-2 is a UPLC chromatogram comparing peak times of rotigotine behenate compound (peak No. 2) and (*S*)-6-(propylamino)-5,6,7,8-tetrahydronaphthalene-1-yl didodecanoate (peak No. 1).
FIG. 5 is a high-resolution mass spectrogram of 7,8-dihydronaphthalene-1-yl didodecanoate.
FIG. 6-1 is a UPLC chromatogram of a reference sample of 7,8-dihydronaphthalene-1-yl didodecanoate.
FIG. 6-2 is a UPLC chromatogram comparing peak times of rotigotine behenate compound (peak No. 2) and 7,8-dihydronaphthalene-1-yl didodecanoate (peak No. 1).
FIG. 7 is a UPLC chromatogram of detecting (*S*)-6-(2-(2-thienyl)ethyl)amino)-5,6,7,8-tetrahydronaphthalene-1-yl didodecanoate (peak No. 3) in rotigotine behenate.
FIG. 8 is a UPLC chromatogram of detecting (*S*)-6-(propylamino)-5,6,7,8-tetrahydronaphthalene-1-yl didodecanoate (peak No. 1) in rotigotine behenate.
FIG. 9 is a UPLC chromatogram of detecting 7,8-dihydronaphthalene-1-yl didodecanoate (peak No. 3) in rotigotine behenate.

### DETAILED DESCRIPTION

The present invention will be further illustrated below by examples and experimental examples, but is not limited in any way.

### Example 1: Preparation of

### (S)-6-(2-(2-thienyl)ethyl)amino)-5,6,7,8-tetrahydronaphthalene-1-yl didodecanoate

20.98 g of (*S*)-6-(2-(thiophene-2-yl)ethylamino)-5,6,7,8-tetrahydronaphthalene-1-alcohol hydrobromide, 14.98 g of triethylamine and 210 mL of dichloromethane were added into a 500 mL single-neck flask and stirred at room temperature;
15.51 g of di-*tert*-butyl dicarbonate was slowly added dropwise, the resulting mixture was stirred overnight for 17 h at room temperature after the addition was complete, and the starting materials were consumed completely as detected by TLC;
the solvent was evaporated off under vacuum, stirring was performed with silica gel, and column chromatography was performed to give 10.66 g of intermediate **I**.
14.0 g of intermediate **I**, 14.05 g of behenic acid, 5.45 g of DMAP, 9.35 g of EDCI and 210 mL of toluene were weighed and added into a 1000 mL single-neck flask and uniformly stirred to react at 40 °C for 3 h, and the starting materials were consumed completely as detected by TLC;
210 mL of 20% NH₄Cl solution was added and stirred for 0.5 h, diatomite was added followed by suction filtration, the solution was allowed to stand overnight, the organic phase was washed once with 190 mL of 20% NH₄Cl solution, the organic phase was added with water and stirred for 0.5 h, and after the solution was allowed to stand for 1 h, the aqueous phase was removed;
the organic phase solvent was evaporated off, and column chromatography was performed to give 225.4 g of an intermediate;
218.8 g of the intermediate was added into a 500 mL three-neck flask and dissolved in 190 mL of dichloromethane, the solution was added with 21.8 g of *N*-methylmorpholine and uniformly stirred, the solution was then added with 21.6 g of TMSI and stirred at room temperature to react for 2 h, and the starting materials were consumed completely as detected by TLC;
the organic solvent was evaporated off under vacuum (60 °C, 0.5 h), 200 mL of petroleum ether was added, the resulting mixture was stirred for 15 min followed by suction filtration, and the filter cake was discarded;
the mother solution was concentrated and evaporated, 150 mL of dichloromethane was added, and dichloromethane was washed with 150 mL of water twice;
the organic phase was concentrated and evaporated, and after 2 h of drying by blowing at 40 °C, 14.1 g of (*S*)-6-(2-(2-thienyl)ethyl)amino)-5,6,7,8-tetrahydronaphthalene-1-yl didodecanoate was given.

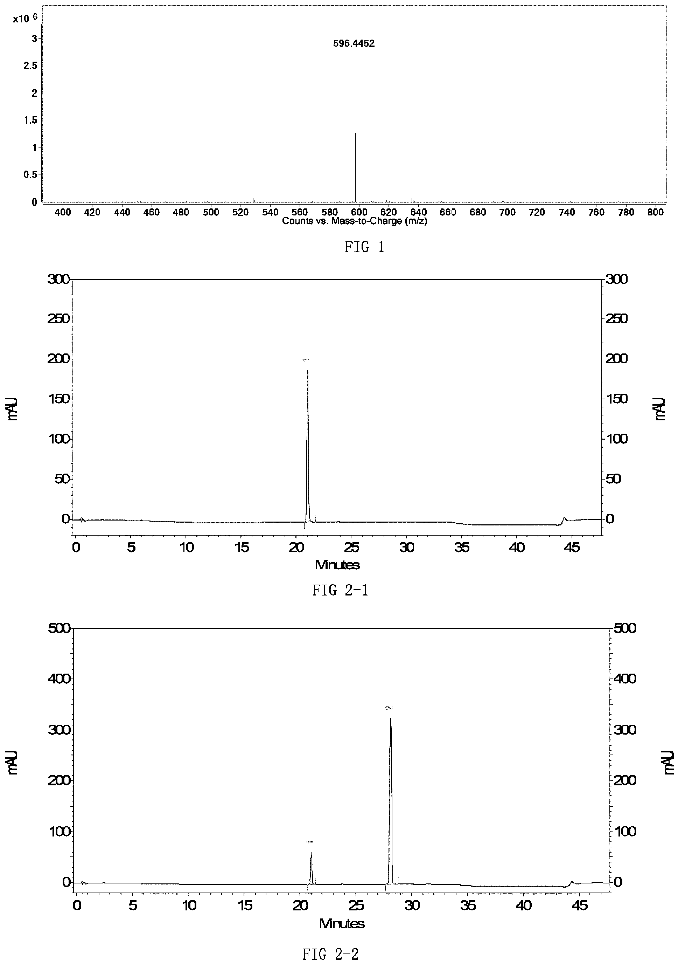

An assignment of the nuclear magnetic resonance spectrum is shown in Table 1, and the high-resolution mass spectrogram is shown in FIG. 1.

**Table 1: Assignment of Nuclear Magnetic Resonance Spectrum of (S)-6-(2-(2-thienyl)ethyl)amino)-5,6,7,8-tetrahydronaphthalene-1-yl didodecanoate**

| NO | ¹H NMR | ¹³C NMR | NO | ¹H NMR | ¹³C NMR |
|---|---|---|---|---|---|
| 1 | / | 148.92 | 1" | / | 172.06 |
| 2 | 6.936 | 119.29 | 2" | 2.598 | 36.70 |
| 3 | 7.117 | 126.98 | 3" | 1.424 | 25.09 |
| 4 | 6.965 | 123.58 | 4" | 1.283 | 28.94 |
| 5 | 3.017,2.953 | 34.229 | 5" | 1.283 | 29.21 |
| 6 | 2.735 | 52.75 | 6" | 1.283 | 29.67 |
| 7 | 1.555,1.770 | 29.71 | 7" | 1.283 | 29.67 |
| 8 | 2.565,2.614 | 22.38 | 8" | 1.283 | 29.67 |
| 9 | / | 128.63 | 9" | 1.283 | 29.67 |
| 10 | / | 137.38 | 10" | 1.283 | 29.67 |
| 1' | 3.057 | 48.36 | 11" | 1.283 | 29.67 |
| 2' | 3.038 | 30.70 | 12" | 1.283 | 29.67 |
| 3' | / | 142.54 | 13" | 1.283 | 29.67 |
| 4' | 6.936 | 126.34 | 14" | 1.283 | 29.67 |
| 5' | 6.834 | 126.84 | 15" | 1.283 | 29.67 |
| 6' | 7.153 | 125.03 | 16" | 1.283 | 29.67 |
| | | | 17" | 1.283 | 29.67 |
| | | | 18" | 1.283 | 29.67 |
| | | | 19" | 1.283 | 29.27 |
| | | | 20" | 1.283 | 31.94 |
| | | | 21" | 1.424 | 22.70 |
| | | | 22" | 0.891 | 14.13 |

The nuclear magnetic resonance structure is numbered as follows:

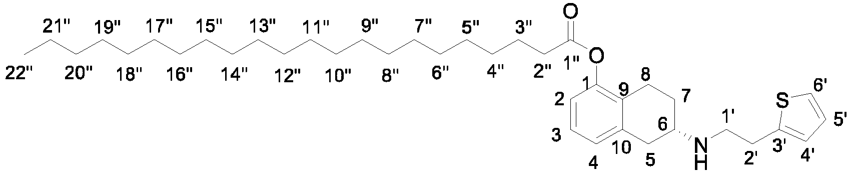

### Experimental Example 1: Use of (S)-6-(2-(2-thienyl)ethyl)amino)-5,6,7,8-tetrahydronaphthalene-1-yl didodecanoate as Reference Sample in Determination of Impurity Content of Rotigotine Behenate Compound

### Sample Preparation:

An appropriate amount of rotigotine behenate was weighed and dissolved in tetrahydrofuran to give a solution containing about 1 mg per 1 mL as a test sample solution; and an appropriate amount of (*S*)-6-(2-(2-thienyl)ethyl)amino)*-*5,6,7,8-tetrahydronaphthalene-1-yl didodecanoate was weighed precisely, dissolved in tetrahydrofuran and diluted quantitatively to give a solution containing about 0.008 mg per 1 mL as a reference sample solution.

### Chromatographic conditions are as follows:

Octadecyl-bonded silica gel was used as a filler for a chromatographic column (XBridge BEH C18 2.1 × 100 mm, 1.7 µm, Waters); gradient elution was performed with 20 mmol/L ammonium acetate solution (1.54 g of ammonium acetate was diluted to 1000 mL with water, and pH value was regulated to 9.0 with ammonia water) with pH of 9.0 as mobile phase A, acetonitrile as mobile phase B and isopropanol as mobile phase C according to the following table; the column temperature was 45 °C; the flow rate was 0.32 mL/min, the detection wavelength was 235 nm, and the number of theoretical plates was not less than 5000 based on rotigotine behenate. 3 µL of the reference sample solution or the test sample solution was injected into an ultra-high performance liquid chromatograph.

### Mobile Phase Elution Gradient

| Time (min) | A(%) | B(%) | C(%) |
|---|---|---|---|
| 0 | 80 | 20 | 0 |
| 10 | 15 | 45 | 40 |
| 26 | 10 | 40 | 50 |
| 32 | 10 | 40 | 50 |
| 32.2 | 0 | 40 | 60 |
| 42 | 0 | 40 | 60 |
| 43 | 80 | 20 | 0 |
| 48 | 80 | 20 | 0 |

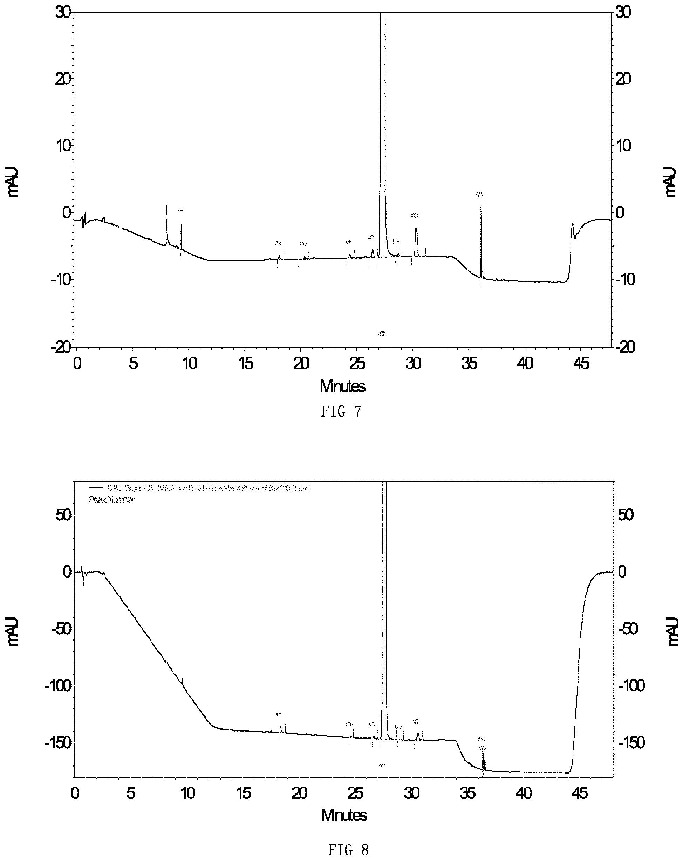

FIG. 2-1 is a UPLC chromatogram of (*S*)-6-(2-(2-thienyl)ethyl)amino)-5,6,7,8-tetrahydronaphthalene-1-yl didodecanoate injected alone. FIG. 2-2 is a UPLC chromatogram comparing peak times of injected rotigotine behenate and added (*S*)-6-(2-(2-thienyl)ethyl)amino)-5,6,7,8-tetrahydronaphthalene-1-yl didodecanoate. FIG. 7 is a UPLC chromatogram of detecting (*S*)-6-(2-(2-thienyl)ethyl)amino)-5,6,7,8-tetrahydronaphthalene-1-yl didodecanoate (peak No. 3) in the rotigotine behenate product. Calculated by an external standard method, the contents of (*S*)-6-(2-(2-thienyl)ethyl)amino)-5,6,7,8-tetrahydronaphthalene-1-yl didodecanoate in three batches of rotigotine behenate compounds were below 0.5%.

### Experimental Example 2: Use of (S)-6-(2-(2-thienyl)ethyl)amino)-5,6,7,8-tetrahydronaphthalene-1-yl didodecanoate as Reference Sample in Determination of Impurity Content of Rotigotine Microsphere Preparation

An appropriate number of sustained release rotigotine behenate microspheres (approximately equivalent to 10 mg of rotigotine behenate) were precisely weighed and dissolved in tetrahydrofuran to give a solution containing about 1 mg per 1 mL as a test sample solution; and an appropriate amount of (*S*)-6-(2-(2-thienyl)ethyl)amino)-5,6,7,8-tetrahydronaphthalene-1-yl didodecanoate was weighed precisely, dissolved in tetrahydrofuran and diluted quantitatively to give a solution containing about 0.008 mg per 1 mL as a reference sample solution. Chromatographic conditions are as follows:
Octadecyl-bonded silica gel was used as a filler for a chromatographic column (XBridge BEH C18 2.1 × 100 mm, 1.7 µm, Waters); gradient elution was performed with 20 mmol/L ammonium acetate solution (1.54 g of ammonium acetate was diluted to 1000 mL with water, and pH value was regulated to 9.0 with ammonia water) with pH of 9.0 as mobile phase A, acetonitrile as mobile phase B and isopropanol as mobile phase C according to the following table; the column temperature was 45 °C; the flow rate was 0.32 mL/min, the detection wavelength was 235 nm, and the number of theoretical plates was not less than 5000 based on rotigotine behenate. 3 µL of the reference sample solution or the test sample solution was injected into an ultra-high performance liquid chromatograph.

### Mobile Phase Elution Gradient

| Time (min) | A(%) | B(%) ) | C(%) |
|---|---|---|---|
| 0 | 80 | 20 | 0 |
| 10 | 15 | 45 | 40 |
| 26 | 10 | 40 | 50 |
| 32 | 10 | 40 | 50 |
| 32.2 | 0 | 40 | 60 |
| 42 | 0 | 40 | 60 |
| 43 | 80 | 20 | 0 |
| 48 | 80 | 20 | 0 |

Calculated by an external standard method, the weight percentages of (*S*)-6-(2-(2-thienyl)ethyl) amino)-5,6,7,8-tetrahydronaphthalene-1-yl didodecanoate in three batches of rotigotine behenate microspheres were below 0.5%.

### Example 2: Preparation of (S)-6-(propylamino)-5,6,7,8-tetrahydronaphthalene-1-yl didodecanoate

26.4 g of (*S*)-6-(propylamino)-5,6,7,8-tetrahydronaphthalene-1-alcohol hydrobromide, 21.46 g of triethylamine and 260 mL of dichloromethane were weighed and stirred at room temperature; 26.16 g of di-*tert*-butyl dicarbonate was slowly added dropwise, the resulting mixture was stirred at room temperature for 4 h after the addition was complete, and the starting materials were consumed completely as detected by TLC; the solvent was evaporated off under vacuum, and column chromatography was performed to give 24.1 g of intermediate I;
24.1 g of intermediate I, 29.56 g of behenic acid, 11.47 g of DMAP, 19.66 g of EDCI and 350 mL of toluene were weighed and uniformly stirred to react at 40 °C for 3 h, and the starting materials were consumed completely as detected by TLC; 360 mL of 20% NH₄Cl solution was stirred for 0.5 h, diatomite was added followed by suction filtration, the solution was allowed to stand overnight, and the organic phase was washed once with 360 mL of 20% NH₄Cl solution, added with water, stirred for 0.5 h and allowed to stand; the organic phase solvent was evaporated off, and column chromatography was performed to give 248.0 g of an intermediate;
212.56 g of the intermediate was weighed, dissolved in 130 mL of dichloromethane, added with 16.18 g of *N*-methylmorpholine and uniformly stirred, 16.0 g of TMSI was then added, the resulting mixture was stirred at room temperature for 2 h, and the starting materials were consumed completely as detected by TLC; the organic solvent was evaporated off under vacuum (60 °C, 0.5 h), 150 mL of petroleum ether was added, the resulting mixture was stirred for 15 min followed by suction filtration, and the filter cake was discarded; the mother solution was concentrated and evaporated, 150 mL of dichloromethane was added, and dichloromethane was washed twice with 150 mL of water; the organic phase was concentrated and evaporated, and after 2 h of drying by blowing at 40 °C, 7.3 g of (*S*)-6-(propylamino)-5,6,7,8-tetrahydronaphthalene-1-yl didodecanoate was given.

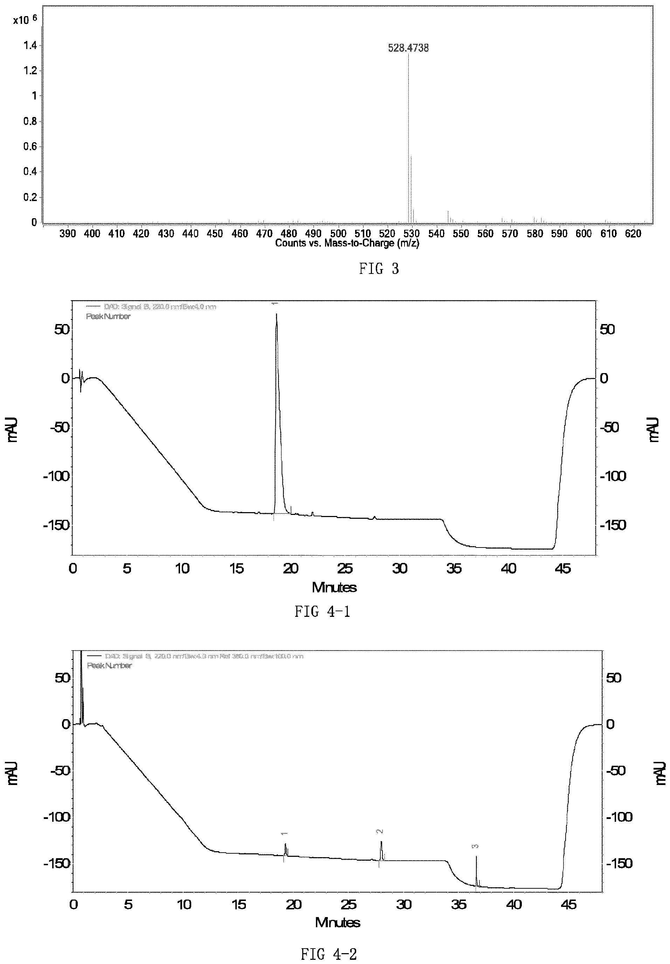

An assignment of the nuclear magnetic resonance spectrum is shown in Table 2, and the high-resolution mass spectrogram is shown in FIG. 3.

**Table 2: Assignment of Nuclear Magnetic Resonance Spectrum of (S)-6-(propylamino)-5,6,7,8-tetrahydronaphthalene-1-yl didodecanoate**

| NO | ¹H NMR | NO | ¹H NMR |
|---|---|---|---|
| 1 | / | 1" | / |
| 2 | 6.832 | 2" | 2.552 |
| 3 | 7.117 | 3" | 1.560 |
| 4 | 6.973 | 4" | 1.252 |
| 5 | 3.050,2.604 | 5" | 1.252 |
| 6 | 2.903 | 6" | 1.252 |
| 7 | 1.782,1.522 | 7" | 1.252 |
| 8 | 2.916,2.535 | 8" | 1.252 |
| 9 | / | 9" | 1.252 |
| 10 | / | 10" | 1.252 |
| 1' | 2.668 | 11" | 1.252 |
| 2' | 1.423 | 12" | 1.252 |
| 3' | 0.950 | 13" | 1.252 |
| | | 14" | 1.252 |
| | | 15" | 1.252 |
| | | 16" | 1.252 |
| | | 17" | 1.252 |
| | | 18" | 1.252 |
| | | 19" | 1.252 |
| | | 20" | 1.252 |
| | | 21" | 1.423 |
| | | 22" | 0.887 |

The nuclear magnetic resonance structure is numbered as follows:

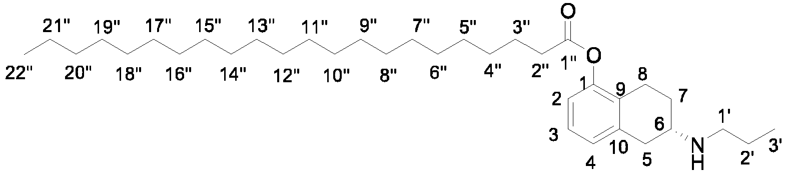

### Experimental Example 3: Use of (S)-6-(propylamino)-5,6,7,8-tetrahydronaphthalene-1-yl didodecanoate as Reference Sample in Determination of Impurity Content of Rotigotine Behenate Compound

### Sample Preparation:

An appropriate amount of rotigotine behenate was weighed and dissolved in tetrahydrofuran to give a solution containing about 1 mg per 1 mL as a test sample solution; and an appropriate amount of (*S*)-6-(propylamino)-5,6,7,8-tetrahydronaphthalene-1-yl didodecanoate was weighed precisely, dissolved in tetrahydrofuran and diluted quantitatively to give a solution containing about 0.005 mg per 1 mL as a reference sample solution.

### Chromatographic conditions are as follows:

Octadecyl-bonded silica gel was used as a filler for a chromatographic column (XBridge BEH C18 2.1 × 100 mm, 1.7 µm, Waters); gradient elution was performed with 20 mmol/L ammonium acetate solution (1.54 g of ammonium acetate was diluted to 1000 mL with water, and pH value was regulated to 9.0 with ammonia water) with pH of 9.0 as mobile phase A, acetonitrile as mobile phase B and isopropanol as mobile phase C according to the following table; the column temperature was 45 °C; the flow rate was 0.32 mL/min, the detection wavelength was 220 nm, and the number of theoretical plates was not less than 5000 based on rotigotine behenate. 3 µL of the reference sample solution or the test sample solution was injected into an ultra-high performance liquid chromatograph.

### Mobile Phase Gradient

| Time (min) | A(%) | B(%) | C(%) |
|---|---|---|---|
| 0 | 80 | 20 | 0 |
| 10 | 15 | 45 | 40 |
| 26 | 10 | 40 | 50 |
| 32 | 10 | 40 | 50 |
| 32.2 | 0 | 40 | 60 |
| 42 | 0 | 40 | 60 |
| 43 | 80 | 20 | 0 |
| 48 | 80 | 20 | 0 |

FIG. 4-1 is a UPLC chromatogram of (*S*)-6-(propylamino)-5,6,7,8-tetrahydronaphthalene-1-yl didodecanoate injected alone. FIG. 4-2 is a UPLC chromatogram comparing peak times of injected rotigotine behenate and added (*S*)-6-(propylamino)-5,6,7,8-tetrahydronaphthalene-1-yl didodecanoate. FIG. 8 is a UPLC chromatogram of detecting (*S*)-6-(propylamino)-5,6,7,8-tetrahydronaphthalene-1-yl didodecanoate (peak No. 1) in the rotigotine behenate product. Calculated by an external standard method, the contents of (*S*)-6-(propylamino)-5,6,7,8-tetrahydronaphthalene-1-yl didodecanoate in three batches of rotigotine behenate compounds were below 0.5%.

### Experimental Example 4: Use of (S)-6-(propylamino)-5,6,7,8-tetrahydronaphthalene-1-yl didodecanoate as Reference Sample in Determination of Impurity Content of Rotigotine Behenate Microsphere Preparation

### Sample Preparation:

An appropriate number of sustained release rotigotine behenate microspheres (approximately equivalent to 10 mg of rotigotine behenate) were precisely weighed, poured into a 10 mL measuring flask and dissolved in 10 mL of tetrahydrofuran to give a test sample solution; and an appropriate amount of (*S*)-6-(propylamino)-5,6,7,8-tetrahydronaphthalene-1-yl didodecanoate was weighed precisely and diluted quantitatively with tetrahydrofuran to give a solution containing about 0.005 mg per 1 mL as a reference sample solution.

### Chromatographic conditions are as follows:

Octadecyl-bonded silica gel was used as a filler for a chromatographic column (XBridge BEH C18 2.1 × 100 mm, 1.7 µm, Waters); gradient elution was performed with 20 mmol/L ammonium acetate solution (1.54 g of ammonium acetate was diluted to 1000 mL with water, and pH value was regulated to 9.0 with ammonia water) with pH of 9.0 as mobile phase A, acetonitrile as mobile phase B and isopropanol as mobile phase C according to the following table; the column temperature was 45 °C, the flow rate was 0.32 mL/min, the detection wavelength was 220 nm, and the number of theoretical plates was not less than 5000 based on rotigotine behenate. 3 µL of the reference sample solution or the test sample solution was injected into an ultra-high performance liquid chromatograph.

### Mobile Phase Elution Gradient

| Time (min) | A(%) | B(%) | C(%) |
|---|---|---|---|
| 0 | 80 | 20 | 0 |
| 10 | 15 | 45 | 40 |
| 26 | 10 | 40 | 50 |
| 32 | 10 | 40 | 50 |
| 32.2 | 0 | 40 | 60 |
| 42 | 0 | 40 | 60 |
| 43 | 80 | 20 | 0 |
| 48 | 80 | 20 | 0 |

Calculated by an external standard method, the weight percentages of (*S*)-6-(propylamino)-5,6,7,8-tetrahydronaphthalene-1-yl didodecanoate in three batches of rotigotine behenate microspheres were below 0.5%.

### Example 3: Preparation of 7,8-dihydronaphthalene-1-yl didodecanoate

93 g of 5-hydroxy-1-tetralone, 103 g of imidazole and 106 g of TBS-Cl were added into 500 mL of DMF to form a light brown yellow solution, a white solid was precipitated from the reaction solution after 20 min, the reaction was continued for 1 h, and the starting materials were consumed completely as detected by TLC; the reaction solution was poured into 3 L of water to precipitate a large amount of white solid, filtration was performed after 10 min of stirring, the filter cake was washed with water, dissolved in 500 mL of dichloromethane, washed with water, dried and added with petroleum ether, the solution passed through a 100 g silica gel pad (PE : DCM = 2 : 1) to give 5-(*tert*-butyldimethylsiloxy)-3,4-dihydronaphthalene-1(2*H*)-one, 185 g of which was dissolved in 800 mL of tetrahydrofuran to give a light yellow solution, and 800 mL of methanol was added; the temperature of ice water was controlled at 0 °C, sodium borohydride was added in batches, and 1 h later, 27 g of sodium borohydride (with a maximum temperature of 22 °C) was added in total to react completely; the reaction solution was concentrated, and the organic solvent was removed; after removal of the organic solvent, the product was extracted with ethyl acetate (400 mL × 2), and after drying and evaporation, 327 g of a light yellow oily substance was given, which was dissolved in dichloromethane as calculated according to 170 g of the product contained therein;
1330 g of dichloromethane solution of 5-(*tert*-butyldimethylsiloxy)-1,2,3,4-tetrahydronaphthalene-1-ol (as calculated according to 150 g of starting material) and 225 g of triethylamine were added to 1.3 L of dichloromethane, the temperature was controlled to be less than 20 °C, and a solution of methylsulphonyl chloride (117.8 g) in dichloromethane (600 mL) was added dropwise; 10 min after the addition was complete, there were remaining starting materials as detected by TLC; after 110 g of methylsulphonyl chloride was supplemented, the reaction solution was concentrated, added with 500 mL of water for dispersion, extracted with *tert*-butyl methyl ether (1 L × 2), dried and concentrated, and silica gel column chromatography was performed to give 45 g of *tert*-butyl-(7,8-dihydronaphthalene-1-yloxy)dimethylsilane; 40 g of *tert*-butyl-(7,8-dihydronaphthalene-1-yloxy)dimethylsilane was added into 500 mL of methanol, 22.7 g of cesium fluoride was added, and the mixture was heated to 60 °C under the protection of nitrogen to react to form a white turbid solution; 1 h later, the starting materials were consumed completely as detected by TLC, the reaction solution was concentrated and evaporated, the product was extracted with DCM, then DCM was evaporated, 55 g of basic alumina was added, and a sample was stirred and passed through a 40 g basic alumina column (PE : DCM = 5 : 1) to give 8.3 g of 7,8-dihydronaphthol;
8.3 g of 7,8-dihydronaphthol was added into 150 mL of dichloromethane, 8 g of triethylamine was added, a dichloromethane solution of behenoyl chloride (containing about 19 g of behenoyl chloride) was added dropwise to react completely, 100 mL of water was added into the reaction solution, a large amount of solid was given after stirring for a period of time, and filtration was performed to give 10.3 g of an off-white solid, i.e., 7,8-dihydronaphthalene-1-yl didodecanoate.

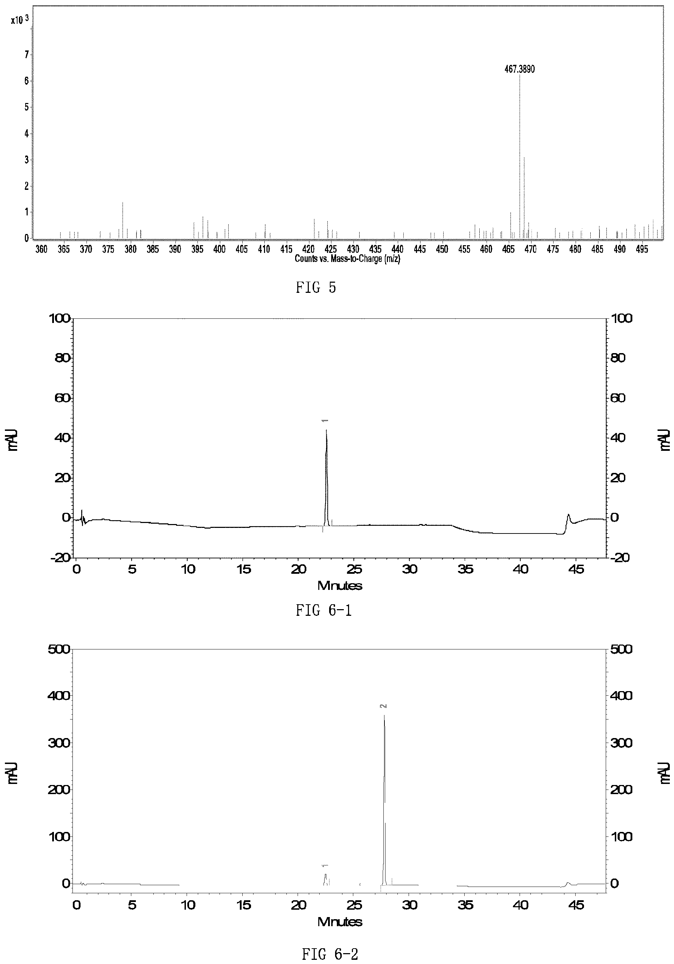

An assignment of the nuclear magnetic resonance spectrum is shown in Table 3, and the high-resolution mass spectrogram is shown in FIG. 5.

**Table 3: Assignment of Nuclear Magnetic Resonance Spectrum of 7,8-dihydronaphthalene-1-yl didodecanoate**

| NO | ¹H NMR | ¹³C NMR | NO | ¹H NMR | ¹³C NMR |
|---|---|---|---|---|---|
| 1 | / | 147.89 | 7' | 1.297 | 29.72 |
| 2 | 6.860 | 120.74 | 8' | 1.297 | 29.72 |
| 3 | 7.152 | 126.77 | 9' | 1.297 | 29.72 |
| 4 | 6.913 | 127.06 | 10' | 1.297 | 29.72 |
| 5 | 6.476 | 123.73 | 11' | 1.297 | 29.72 |
| 6 | 6.049 | 127.42 | 12' | 1.297 | 29.72 |
| 7 | 2.292 | 22.71 | 13' | 1.297 | 29.72 |
| 8 | 2.647 | 20.62 | 14' | 1.297 | 29.72 |
| 9 | / | 135.68 | 15' | 1.297 | 29.72 |
| 10 | / | 128.95 | 16' | 1.297 | 29.72 |
| 1' | / | 172.02 | 17' | 1.297 | 29.72 |
| 2' | 2.569 | 34.26 | 18' | 1.297 | 29.72 |
| 3' | 1.769 | 25.10 | 19' | 1.297 | 29.38 |
| 4' | 1.347 | 29.21 | 20' | 1.297 | 31.94 |
| 5' | 1.323 | 29.27 | 21' | 1.392 | 22.36 |
| 6' | 1.308 | 29.72 | 22' | 0.891 | 14.13 |

The nuclear magnetic resonance structure is numbered as follows:

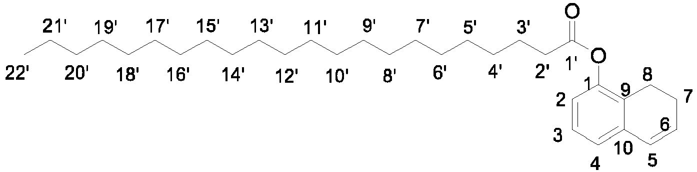

### Experimental Example 5: Use of 7,8-dihydronaphthalene-1-yl didodecanoate as Reference Sample in Determination of Impurity Content of Rotigotine Behenate Compound

### Sample Preparation:

An appropriate amount of rotigotine behenate was weighed and dissolved in tetrahydrofuran to give a solution containing about 1 mg per 1 mL as a test sample solution; and an appropriate amount of 7,8-dihydronaphthalene-1-yl didodecanoate was weighed precisely, dissolved in tetrahydrofuran and diluted quantitatively to give a solution containing about 0.005 mg per 1 mL as a reference sample solution.

### Chromatographic conditions are as follows:

Octadecyl-bonded silica gel was used as a filler for a chromatographic column (XBridge BEH C18 2.1 × 100 mm, 1.7 µm, Waters); gradient elution was performed with 20 mmol/L ammonium acetate solution (1.54 g of ammonium acetate was diluted to 1000 mL with water, and pH value was regulated to 9.0 with ammonia water) with pH of 9.0 as mobile phase A, acetonitrile as mobile phase B and isopropanol as mobile phase C according to the following table; the column temperature was 45 °C; the flow rate was 0.32 mL/min, the detection wavelength was 235 nm, and the number of theoretical plates was not less than 5000 based on rotigotine behenate. 3 µL of the reference sample solution or the test sample solution was injected into an ultra-high performance liquid chromatograph.

### Mobile Phase Elution Gradient

| Time (min) | A(%) | B(%) | C(%) |
|---|---|---|---|
| 0 | 80 | 20 | 0 |
| 10 | 15 | 45 | 40 |
| 26 | 10 | 40 | 50 |
| 32 | 10 | 40 | 50 |
| 32.2 | 0 | 40 | 60 |
| 42 | 0 | 40 | 60 |
| 43 | 80 | 20 | 0 |
| 48 | 80 | 20 | 0 |

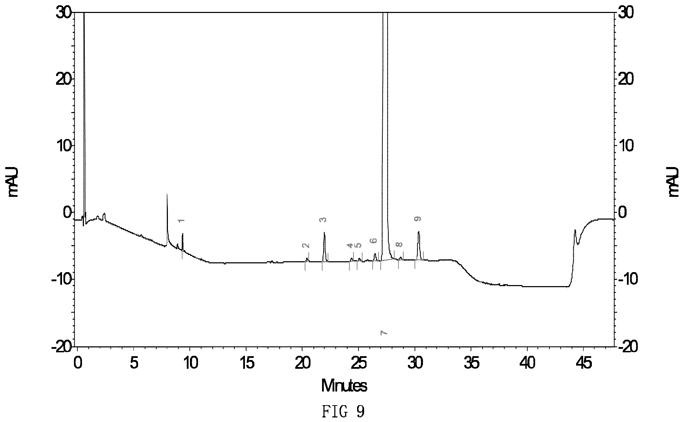

FIG. 6-1 is a UPLC chromatogram of a reference sample of 7,8-dihydronaphthalene-1-yl didodecanoate injected alone. FIG. 6-2 is a UPLC chromatogram comparing peak times of injected rotigotine behenate and 7,8-dihydronaphthalene-1-yl didodecanoate. FIG. 9 is a UPLC chromatogram of detecting 7,8-dihydronaphthalene-1-yl didodecanoate (peak No. 3) in the rotigotine behenate product. Calculated by an external standard method, the contents of 7,8-dihydronaphthalene-1-yl didodecanoate in three batches of rotigotine behenate compounds were below 0.5%.

### Experimental Example 6: Use of 7,8-dihydronaphthalene-1-yl didodecanoate as Reference Sample in Determination of Impurity Content of Rotigotine Behenate Microsphere Preparation

### Sample Preparation:

An appropriate number of sustained release rotigotine behenate microspheres (approximately equivalent to 10 mg of rotigotine behenate) were precisely weighed, poured into a 10 mL measuring flask and dissolved in 10 mL of tetrahydrofuran to give a test sample solution; and an appropriate amount of 7,8-dihydronaphthalene-1-yl didodecanoate was weighed precisely and diluted quantitatively with tetrahydrofuran to give a solution containing about 0.005 mg per 1 mL as a reference sample solution.

### Chromatographic conditions are as follows:

Octadecyl-bonded silica gel was used as a filler for a chromatographic column (XBridge BEH C18 2.1 × 100 mm, 1.7 µm, Waters); gradient elution was performed with 20 mmol/L ammonium acetate solution (1.54 g of ammonium acetate was diluted to 1000 mL with water, and pH value was regulated to 9.0 with ammonia water) with pH of 9.0 as mobile phase A, acetonitrile as mobile phase B and isopropanol as mobile phase C according to the following table; the column temperature was 45 °C; the flow rate was 0.32 mL/min, the detection wavelength was 235 nm, and the number of theoretical plates was not less than 5000 based on rotigotine behenate. 3 µL of the reference sample solution or the test sample solution was injected into an ultra-high performance liquid chromatograph.

### Mobile Phase Elution Gradient

| Time (min) | A(%) | B(%) | C(%) |
|---|---|---|---|
| 0 | 80 | 20 | 0 |
| 10 | 15 | 45 | 40 |
| 26 | 10 | 40 | 50 |
| 32 | 10 | 40 | 50 |
| 32.2 | 0 | 40 | 60 |
| 42 | 0 | 40 | 60 |
| 43 | 80 | 20 | 0 |
| 48 | 80 | 20 | 0 |

Calculated by an external standard method, the weight percentages of 7,8-dihydronaphthalene-1-yl didodecanoate in three batches of rotigotine behenate microspheres were below 0.5%.

## Claims

1. A compound having a structural formula shown as formula I:

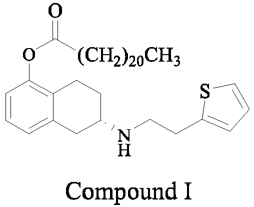

2. A compound having a structural formula shown as formula II:

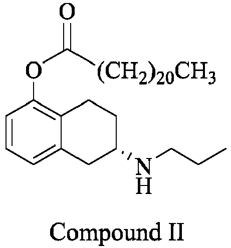

3. A compound having a structural formula shown as formula III:

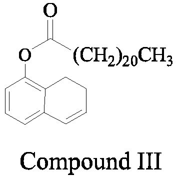

4. A preparation method for the compound according to claim 1, comprising the following steps: weighing (*S*)-6-(2-(thiophene-2-yl)ethylamino)-5,6,7,8-tetrahydronaphthalene-1-alcohol hydrobromide, triethylamine, and dichloromethane, and stirring the mixture at room temperature; adding di-tert-butyl dicarbonate to react completely, and evaporating off the solvent under vacuum to give an intermediate I; weighing the intermediate I, behenic acid, DMAP, EDCI, and toluene, and uniformly stirring; after the reaction is complete, adding an NH₄Cl solution, stirring, performing suction filtration, allowing the solution to stand overnight, washing the solution with an NH₄Cl solution once, adding water, stirring, and after allowing the solution to stand, removing the aqueous phase; after the organic phase solvent is evaporated off, performing column chromatography to give an intermediate II; weighing and dissolving the intermediate II in dichloromethane, adding *N*-methylmorpholine, uniformly stirring, then adding TMSI, and stirring the mixture at room temperature for reaction; after the reaction is complete, evaporating off the organic solvent under vacuum, adding petroleum ether, stirring, performing suction filtration, concentrating and evaporating the mother solution, adding dichloromethane, washing the solution with water; and concentrating, evaporating and drying the organic phase to give a compound I; wherein, preferably, the added NH₄Cl solution is a 20% NH₄Cl solution; preferably, the intermediate I, behenic acid, DMAP, EDCI and toluene are weighed and stirred to react completely at 40 °C; and preferably, di-*tert*-butyl dicarbonate is slowly added dropwise.

5. A preparation method for the compound II according to claim 2, comprising the following steps: weighing (*S*)-6-(propylamino)-5,6,7,8-tetrahydronaphthalene-1-alcohol hydrobromide, triethylamine, and dichloromethane, and stirring the mixture at room temperature; adding di-*tert*-butyl dicarbonate to react completely, and evaporating off the solvent under vacuum to give an intermediate I; weighing the intermediate I, behenic acid, DMAP, EDCI, and toluene, and uniformly stirring; after the reaction is complete, adding an NH₄Cl solution, stirring, performing suction filtration, allowing the solution to stand overnight, washing the solution with an NH₄Cl solution once, adding water, stirring, and after allowing the solution to stand, removing the aqueous phase; after the organic phase solvent is evaporated off, performing column chromatography to give an intermediate II; weighing and dissolving the intermediate II in dichloromethane, adding *N*-methylmorpholine, uniformly stirring, then adding TMSI, and stirring the mixture at room temperature for reaction; after the reaction is complete, evaporating off the organic solvent under vacuum, adding petroleum ether, stirring, performing suction filtration, concentrating and evaporating the mother solution, adding dichloromethane, and washing the solution with water; and concentrating, evaporating and drying the organic phase to give a compound II; wherein, preferably, the NH₄Cl solution added by the method is a 20% NH₄Cl solution; preferably, in the method, the intermediate I, behenic acid, DMAP, EDCI and toluene are weighed and stirred to react completely at 40 °C; and preferably, di-*tert*-butyl dicarbonate is slowly added dropwise.

6. A preparation method for the compound III according to claim 3, comprising the following steps: adding 5-hydroxy-1-tetralone, imidazole and TBS-Cl into DMF to form a light brown yellow solution, and reacting completely; pouring the reaction solution into water, precipitating a solid, performing filtration, washing the filter cake with water, then dissolving the filter cake in dichloromethane, washing the solution with water, adding petroleum ether after drying, passing through a silica gel pad to give 5-(*tert*-butyldimethylsiloxy)-3,4-dihydronaphthalene-1(2*H*)-one, dissolving 5-(*tert*-butyldimethylsiloxy)-3,4-dihydronaphthalene-1(2*H*)-one in tetrahydrofuran, adding methanol, adding sodium borohydride in batches, and reacting completely; concentrating the reaction solution, removing the organic solvent, extracting the product with ethyl acetate to give a light yellow oily substance, dissolving the light yellow oily substance in dichloromethane, adding a dichloromethane solution of 5-(*tert*-butyldimethylsiloxy)-1,2,3,4-tetrahydronaphthalene-1-ol and triethylamine into dichloromethane, adding a dichloromethane solution of methylsulphonyl chloride dropwise while temperature is controlled, concentrating the reaction solution, adding water for dispersion, extracting the solution with *tert*-butyl methyl ether, concentrating the solution after drying, and performing silica gel column chromatography to give *tert*-butyl-(7,8-dihydronaphthalene-1-yloxy) dimethylsilane; and adding *tert*-butyl-(7,8-dihydronaphthalene-1-yloxy)dimethylsilane into methanol, adding cesium fluoride, reacting under the protection of nitrogen, concentrating the reaction solution after the reaction is complete, extracting the product with DCM after evaporation, then evaporating DCM, adding basic alumina, stirring a sample, passing through an basic alumina column (PE : DCM = 5 : 1) to give 7,8-dihydronaphthol, adding 7,8-dihydronaphthol into dichloromethane, adding triethylamine and a dichloromethane solution of behenoyl chloride, reacting completely, adding water into the reaction solution, stirring, and performing filtration to give the compound III; wherein, preferably, the reaction under the protection of nitrogen is performed by heating to 60 °C; preferably, the dichloromethane solution of 5-(*tert*-butyldimethylsiloxy)-1,2,3,4-tetrahydronaphthalene-1-ol and triethylamine are added into dichloromethane, and the temperature is controlled to be less than 20 °C.

7. Use of the compound according to any of claims 1 to 3 as a reference sample in the detection of impurities in rotigotine behenate or a preparation thereof.

8. A method for determining the impurity content of rotigotine behenate or a preparation thereof, employing liquid chromatography for analysis, wherein the compound according to any of claims 1 to 3 is used as a reference sample.

9. The method according to claim 8, wherein the compound according to any of claims 1 to 3 is dissolved in a solution to prepare a reference sample solution, and rotigotine behenate or a preparation thereof is dissolved in a solution to prepare a test sample solution; analysis is performed by employing liquid chromatography to give a liquid chromatogram of the reference sample solution and the test sample solution; the peak times in the liquid chromatogram of the reference sample solution and the test sample solution are compared, it is determined that the test sample solution contains the compound according to any of claims 1 to 3, and the weight percentage of the compound according to any of claims 1 to 3 in the rotigotine behenate or the preparation thereof is determined according to an external standard method.

10. A rotigotine behenate or a preparation thereof, containing less than 0.5 wt% of the compound according to any of claims 1 to 3.
